**Europäisches Patentamt**

**European Patent Office**

**Office européen des brevets**

(19)

(11) Veröffentlichungsnummer: **0 019 168**
**B1**

(12) **EUROPÄISCHE PATENTSCHRIFT**

(45) Veröffentlichungstag der Patentschrift:
**08.06.83**

(51) Int. Cl.³: **A 01 M 5/00,** A 01 K 67/00

(21) Anmeldenummer: **80102410.0**

(22) Anmeldetag: **03.05.80**

(54) **Verwendung einer Vorrichtung zum Anlocken von Nutzinsekten.**

(30) Priorität: **10.05.79 DE 2918852**

(43) Veröffentlichungstag der Anmeldung:
**26.11.80 Patentblatt 80/24**

(45) Bekanntmachung des Hinweises auf die Patenterteilung:
**08.06.83 Patentblatt 83/23**

(84) Benannte Vertragsstaaten:
**AT BE CH DE FR GB IT LI LU NL**

(56) Entgegenhaltungen:

DE-A-2 821 378
DE-C-814 805
FR-A-449 779
GB-A-2 000 007
US-A-1 464 133
US-A-2 080 160
US-A-3 941 089

(73) Patentinhaber: **Bartz, Gisela, Winnekendonker Str. 43,
D-4179 Kevelaer 2 (DE)**

(72) Erfinder: **Bartz, Gisela, Winnekendonker Str. 43,
D-4179 Kevelaer 2 (DE)**

(74) Vertreter: **Stark, Walter, Dr.-Ing., Moerser Strasse 140,
D-4150 Krefeld (DE)**

Verwendung einer Vorrichtung zum Anlocken von Nutzinsekten

Die Erfindung betrifft die Verwendung einer Vorrichtung zum Anlocken von Nutzinsekten.

Behälter mit mehreren Löchern, die teilweise mit Naturstoff gefüllt sind, wie z. B. Vogelkästen, sind bekannt.

Zur Schädlingsbekämpfung in Garten, Feld oder Wald werden meistens Insektizide eingesetzt, mit denen die Nutzpflanzen besprüht werden. Bäume erhalten Leimringe, um sie vor Befall mit schädlichen Insekten zu schützen. Diese Art der Schädlingsbekämpfung ist kosten- und arbeitsaufwendig.

Andererseits sind Nutzinsekten bekannt, deren Nahrung gerade die Schädlinge sind. Zum Beispiel können Nutzinsekten vom Typ «Forficula auri cularia» (Ohrwurm) grosse Mengen von Schädlingen vertilgen und dadurch eine Pflanzenkultur vor Schädlingsbefall schützen. Die genannten Insekten vertilgen z. B. vorzugsweise Blattläuse und Obstmaden.

Bekannt sind sogenannte Insektenfallen, also Vorrichtungen zur Vernichtung von Insekten (DE-C 814 805, US-A 14 64 133). Dabei handelt es sich um Behälter mit Schlupflöchern für die Schädlichen Insekten. Die Behälter sind teilweise mit Lockstoffen oder Naturstoffen gefüllt.

Aufgabe der Erfindung ist es, Nutzinsekten zur Schädlingsbekämpfung einzusetzen.

Diese Aufgabe wird gelöst durch die Verwendung eines Behälters, der mehrere Schlupflöcher für Insekten aufweist und der wenigstens teilweise mit Naturstoffen zur Entwicklung eines von Nutzinsekten bevorzugten Klimas gefüllt ist, für die Schädlingsbekämpfung in Garten, Feld oder Wald mit Hilfe von Nutzinsekten.

Derartige Vorrichtungen können in Beeten, Pflanzungen und dergleichen in mehr oder weniger grosser Zahl aufgestellt oder aufgehängt werden. Sie dienen dann den Nutzinsekten, die nach einem sicheren Platz suchen, als Unterschlupf und veranlassen dadurch die Nutzinsekten, sich in der nähren Umgebung auf Nahrungssuche zu begeben. Da diese Nutzinsekten vorzugsweise Schädlinge angreifen und vertilgen, bleibt die betreffende Pflanzenkultur frei von Schädlingsbefall.

Der Behälter kann insbesondere einen nicht geschlossenen Boden, eine zylindrische oder konisch verjüngte Seitenwand mit mehreren darin angeordneten Schlupflöchern und einen am oberen Ende lösbar befestigten Belüftungs- und Regendachdeckel aufweisen. Gegebenenfalls kann auch eine Aufhängeeinrichtung vorgesehen sein, die beispielsweise ein Bügel ist, der an einem sich durch den Behälter erstreckenden Draht oder dergleichen gehalten ist, wobei der Draht am Boden befestigt ist. Vorzugsweise kann der Draht durch den Boden hindurchgeführt sein und an der Unterseite des Bodens an ein Widerlager angeschlossen sein. All das ermöglicht eine äusserst einfache, nichtsdestoweniger im Hinblick auf den vorgesehenen Zweck aber sehr nützliche Konstruktion, deren Herstellungskosten sehr gering sind.

Die Füllung kann aus Holzwolle, Binsen, Stroh oder dergleichen bestehen, jedenfalls aus einem Material, in dem sich ein von den Nutzinsekten bevorzugtes Klima entwickeln kann.

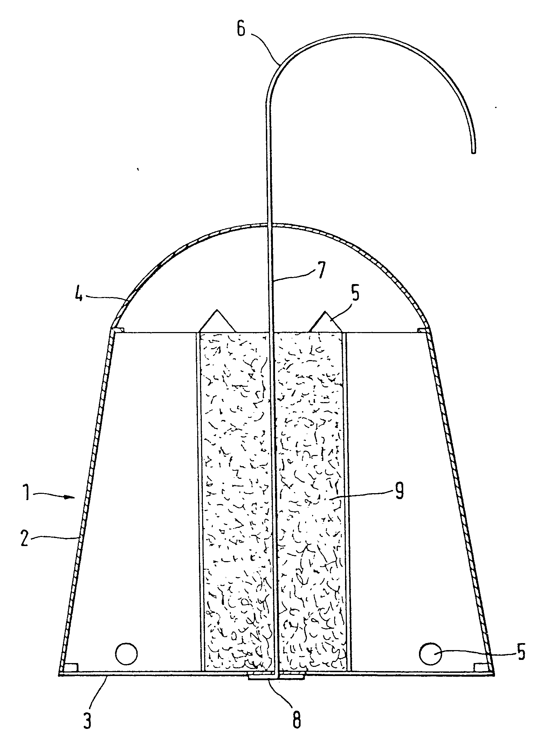

Im folgenden wird ein in der Zeichnung dargestelltes Ausführungsbeispiel der Erfindung erläutert; die einzige Figur zeigt, teilweise geschnitten, eine Seitenansicht einer Vorrichtung zum Anlocken von Nutzinsekten.

Die Vorrichtung besteht in ihrem grundsätzlichen Aufbau aus einem beim dargestellten Ausführungsbeispiel konischen Behälter 1 mit einer Seitenwand 2 aus witterungsbeständigem Material. Der Behälter 1 hat unten einen Boden 3 mit Seitenspalten für den Kotausfall, dessen Material ebenfalls witterungsbeständig ist. Am oberen Ende ist der Behälter 1 mit einem lösbar befestigten, als Regenschutzdienenden Deckel 4 versehen, der beim dargestellten Ausführungsbeispiel halbkugelförmig ausgebildet ist. In der Seitenwandung 2 und im Deckel 4 sind eine Mehrzahl von Schlupflöchern 5 für die anzulockenden Nutzinsekten.

Die dargestellte Vorrichtung besitzt ausserdem eine Aufhängeeinrichtung aus einem Drahtbügel 6, welcher durch den Behälter 1 und durch den Boden 3 geführt ist und unterhalb des Bodens 3 als ein Widerlager 8 gebogen ist, so dass der Behälter am Drahtbügel 6 aufgehängt werden kann. — Der Behälter 1 kann aber auch ohne Aufhängeeinrichtung verwendet werden. Dann wird er auf seinem Boden 3 abgestellt.

Der Behälter ist teilweise mit einer Füllung 9 aus Naturstoffen gefüllt. Bei den Naturstoffen kann es sich um Holzwolle, Binsen, Stroh oder dergleichen handeln, jedenfalls ein Material, in dem sich ein von den Nutzinsekten bevorzugtes Klima entwickeln kann.

## Patentansprüche

1. Verwendung eines Behälters (1), der mehrere Schlupflöcher (5) für Insekten aufweist und der wenigstens teilweise mit Naturstoffen (9) zur Entwicklung eines von Nutzinsekten bevorzugten Klimas gefüllt ist, für die Schädlingsbekämpfung in Garten, Feld oder Wald mit Hilfe von Nutzinsekten.

2. Verwendung nach Anspruch 1, dadurch gekennzeichnet, dass der Behälter (1) einen festen Boden (3) mit Schlitzen, eine zylindrische oder konisch verjüngte Seitenwand (2) mit mehreren darin angeordneten Schlupflöchern (5) und am oberen Ende Schlupf- und Belüftungslöcher sowie eine aufgesteckte Regenschutzkappe (4) aufweist.

3. Verwendung nach Anspruch 1 oder 2, dadurch gekennzeichnet, dass der Behälter eine Aufhängeeinrichtung (6) aufweist.

4. Verwendung nach Anspruch 3, dadurch gekennzeichnet, dass die Aufhängeeinrichtung ein Bügel (6) ist, der an einem sich durch den Behälter (1) erstreckenden Draht (7) oder dergleichen gehalten ist, wobei der Draht (7) am Boden (3) befestigt ist.

5. Verwendung nach Anspruch 4, dadurch gekennzeichnet, dass der Draht (7) durch den Boden (3) hindurchgeführt ist und an der Unterseite des Bodens (3) ein Widerlager bildet.

6. Verwendung nach einem oder mehreren der Ansprche 1-5, dadurch gekennzeichnet, dass die Füllung (9) aus Holzwolle, Binsen, Stroh oder dergleichen besteht.

## Claims

1. Use of a container (1) comprising a plurality of boreholes (5) for insects and being filled at least partially with natural substances (9) for developing a climate preferred by useful insects, for controlling pests in gardens, fields or woods by means of useful insects.

2. Use as claimed in claim 1, characterised in that the container (1) comprises a fixed base (3) with slots, a cylindrical or conically-tapered lateral wall (2) with a plurality of boreholes (5) therein, the upper end of the container having bore or ventilation holes and a mounted waterproof cover (4).

3. Use as claimed in claim 1 or 2, characterised in that the container is provided with a suspension device (6).

4. Use as claimed in claim 3, characterised in that the suspension device is a curved strip (6) retained on a wire (7) or the like extending through the container (1), the wire (7) being secured to the base (3).

5. Use as claimed in claim 4, characterised in that the wire (7) extends through the base (3) and forms an abutment at the lower side of the base (3).

6. Use as claimed in one or more of claims 1-5, characterised in that the filling (9) comprises woodwool, rushes, straw or the like.

## Revendications

1. Utilisation d'un récipient (1) pourvu de plusieurs perçages (5) d'entrée pour des insectes et qui est rempli, au moins en partie, avec des matières naturelles (9) suscettibles de développer un climat préféré par des insectes utiles destinés à combattre et à détruire des insectes nuisibles dans des jardins, champs ou forêts.

2. Utilisation suivant la revendication 1, caracté risée en ce que le récipient (1) comprend un fond fixe (3) pourvu de fentes, une paroi latérale (2) cylin drique ou rétrécie en cône, pourvue de plusieurs per çages d'entrée (5), et, pourvue, à son extrémité su périeure de perçages d'entrée et de ventilation ainsi que d'un couvercle (4) de protection contre la pluie.

3. Utilisation suivant l'une des revendications 1 ou 2, caractérisée en ce que le récipient (1) est pourvu d'un dispositif de suspension (6).

4. Utilisation suivant la revendication 3, caracté risée en ce que le dispositif de suspension est consti tué par un étrier (6) comportant un fil métallique (7) ou un organe analogue, s'étendant à travers le réci pient (1) et fixé au fond (3).

5. Utilisation suivant la revendication 4, caracté risée en ce que le fil métallique (7) traverse le fond (3) et forme sur la face inférieure du fond un palier de butée.

6. Utilisation suivant l'une quelconque des reven dications 1 à 5, caractérisée en ce que la charge de remplissage (9) est constituée par de la sciure de bois, des joncs, ou de la paille, ou analogues.